# EUROPEAN PATENT SPECIFICATION

(11) **EP 2 326 384 B1**
(45) Date of publication and mention of the grant of the patent: **21.12.2011**
(21) Application number: 09786455.7
(22) Date of filing: 25.06.2009
(51) Int. Cl.: A61M 39/24, F16K 15/14

(54) **A CHECK VALVE, IN PARTICULAR FOR MEDICAL APPLICATIONS**
RÜCKSCHLAGVENTIL, INSBESONDERE FÜR MEDIZINISCHE ANWENDUNGEN
CLAPET DE NON-RETOUR, EN PARTICULIER POUR DES APPLICATIONS MÉDICALES

(30) Priority: 10.07.2008 DE 202008009245 U
(43) Date of publication of application: 01.06.2011
(73) Proprietor: Filtertek B.V., County of Limerick (IE)
(72) Inventor: CARMODY, Colm, Co. Kerry (IE)
(74) Representative: Beck, Alexander
(86) International application number: PCT/IB2009/052754
(87) International publication number: WO 2010/004471

(56) References cited:
- EP-A- 1 099 457
- US-A- 4 355 653
- US-A- 4 535 820
- US-A- 4 762 149

## Description

The present invention relates to a check valve, in particular for medical use, comprising a first and a second tube hookup housing and a flexible diaphragm disk which is positioned between said two housings and which, in the event of over-pressure in an intake passage in the first housing may lift off an annular valve seat enclosing a surrounding intake chamber communicating with the intake passage and, in the event of over-pressure in a n outlet passage may be pressed reliably and in minimum time onto the valve seat, the diaphragm disk being fitted at its outer circumference with an annular bulge. Such a check valve is known from an earlier disclosure by applicant, namely from the European patent document 0 612 537.

Check valves of this kind are used in medical practice in the passages of infusion systems, diagnostic equipment, intravenous tube lines and for injection pumps. In relation to the required closing times of low fractions of a second, such valves must close reliably in order to preclude backflow of any possibly contaminated liquids. In medical use, such check valves are exclusively non-return articles, so that simultaneously on one hand their manufacture shall be exceedingly economical and on the other hand statistically exceedingly accurate. Moreover there is a body of explicit, rigorous regulations and tests to assure the required constant operational reliability. In Germany such valves are officially approved in general following rigorous testing by the TÜV (Technical Monitoring Association). These requirements actually are met already in above cited applicant's earlier check valve, namely, in this known design, the annular bulge at the diaphragm circumference in the annular grooves in the mutually opposite tube hookup housings, is implemented in a manner that when assembling the said two housings, radially directed tensile forces are acting on the diaphragm disk. In this manner the diaphragm tension will be adjustable to assure both contact with the valve seat and rapid and safe check-valve response time.
US 4,535,820A, which is considered to represent the most relevant state of the art, discloses a check valve, in particular for medical purposes, comprising a first tube hook up housing and a second tube hook up housing and a diaphragm disk of a flexible plastic which is positioned between said two housings and which in the case of overpressure in an intake passage in the first tube hook up housing is lifted off an annular valve seat enclosing an intake chamber connected to the intake passage and which in the case of overpressure in an outlet passage can be pressed reliably and in minimal time onto the valve seat, wherein the intake chamber is radially substantially enlarged with respect to the intake passage and wherein the valve seat encloses the intake chamber and the diaphragm disc is positioned freely in an annular space between the tube hookup housings and the annular space forms bypass channels radially outside the disk, said bypass channels connecting the intake chamber to an outlet chamber communicating with the outlet passage when the diaphragm disc has been lifted off the valve seat, and the supports are positioned concentrically in the central zone of the outlet chamber and bias the membrane disk toward the valve seat and at least one or more structures are provided at least on the side of the diaphragm disc facing said supports, said structures by engaging the ends of the supports precluding the diaphragm disc from displacing.
Further, US 4,355,653A teaches a diaphragm disc having an annular bulge. However, the bulge in this prior art check valve is to provide a proper valve seat.

The practical use has shown, however, that there is also a need for such a check valve that may operate at very high pressure differentials while also precluding a low pressure gradient at a given flow rate. Accordingly it is the objective of the present invention to create a check valve meeting such requirements and manufactured in very simple and economic manner, moreover assuring the uniform and reliable operation.

With respect to a check valve of the initially cited kind, this objective is essentially attained by the characterizing features of claim 1.

By enlarging the intake chamber, that is, by simultaneously enlarging the diameter of the valve seat enclosing it, the present invention provides a larger flow cross-section when the diaphragm disk has been lifted off said valve seat. The diaphragm disk per se is not significantly clamped in some way between the two tube hookup housings, the required bias toward the valve seat being assured by said supports. At the same time, the quasi positive engagement between said structures and on the diaphragm disk and said supports precludes any displacement of the diaphragm disk within the annular space because these supports enclose the diaphragm disk structures with a certain pressure to generate the required bias.

In a preferred embodiment of the present invention, the supports are positioned circularly and concentrically and the said structures are designed as an integral, ring protruding from the diaphragm disk. In this manner positive engagement in all direction precluding lateral displacement is assured.

As regards an especially preferred embodiment mode of the present invention, identical structures are used on the top and the bottom sides of the diaphragm disk. As a result, automated manufacture of the check valve of the present invention is made substantially simpler because a particualr orientation of the diaphragm disk can be ignored in automated assembly.
In particular, the diaphragm disk inside the annular bulge preferably shall be devoid of any apertures. As a result, the flow cross-section is maximized by radially enlarging the valve seat outward because the bypass channels, which are subtended radially outside the diaphragm disk's annular bulge, now assume the function of the apertures in the known valve's diaphragm disk, hence the entire membrane inside region within the annular bulge is made available.
In particular, the diaphragm disk inside the annular bulge preferably shall be devoid of any apertures. As a result, the flow cross-section is maximized by radially enlarging the valve seat outward because the bypass channels, which are subtended radially outside the diaphragm disk's annular bulge, now assume the function of the apertures in the known valve's diaphragm disk, hence the entire membrane inside region within the annular bulge is made available.

Further it is of advantage too, that the diaphragm disk is relatively thick in the region within the annular bulge. This feature allows that the desired degree of pretension or bias of the diaphragm toward the valve seat can be achieved without requiring additional diaphragm affixation of the diaphragm within the annular space.

The thickness of the diaphragm disk in the zone within the annular bulge preferably is approximately 0.5 to 0.8 times the size of the annular bulge's cross-sectional diameter.

The present invention may be developed further in that the supports ends engaging the diaphragm disk and the structure respectively the ring are designed complementary to the cross-section of the structures. This feature provides especially advantageous positive engagement between the supports and the structure respectively the ring.

Preferably, in this regard, the ring shall have a triangular cross-section and the supports shall be fitted with matching inclined surfaces.

The invention is described in more detail below with reference to the illustrative embodiment shown in the appended drawings.

**Fig. 1** is a sectional perspective view of the check valve of the present invention in its closed state,

**Fig. 2** is a sectional view of the valve of Fig. 1, and

**Fig. 3** sectionally shows, on a larger scale, a view of the central zone of the check valve of Figs. 1 and 2.

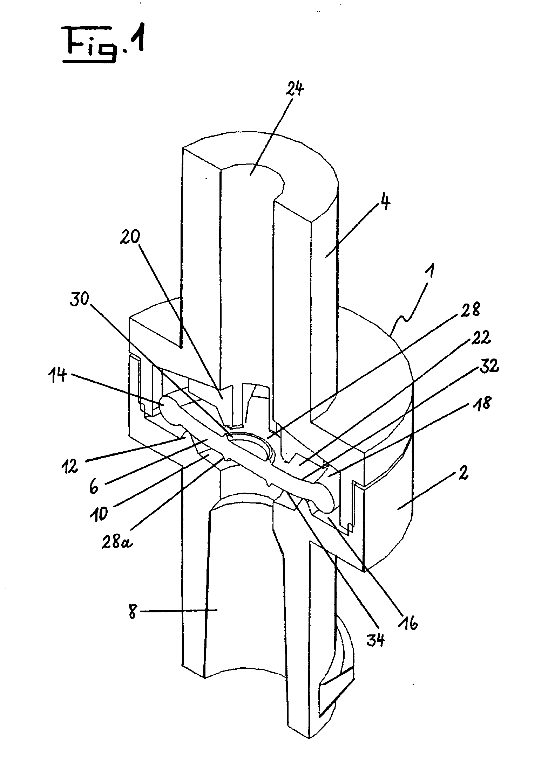

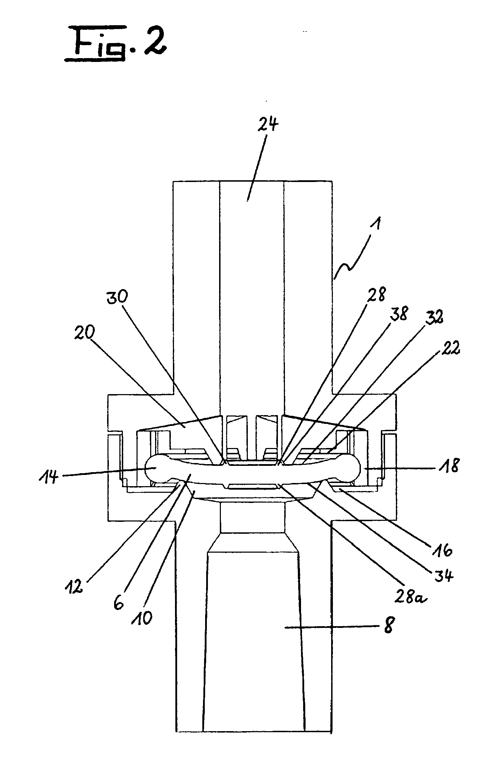

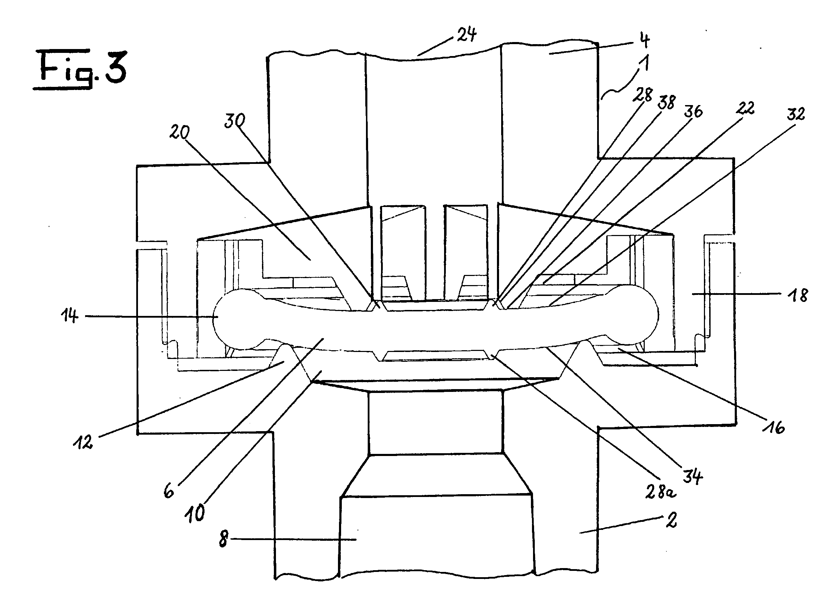

The check valve 1 shown in Figs. 1 through 3 comprises a first tube hookup housing 2 and a second tube hookup housing 4 illustratively made by plastic injection molding. A diaphragm disk 6 illustratively made of a flexible material, such as, silicone, is positioned between the two tube hookup housings 2 and 4.

The first tube hookup housing 2 is fitted with an intake passage 8 opening into an intake chamber 10. This intake chamber 10 is radially considerably larger than the intake passage 8, such that an annular valve seat 12 surrounding the intake chamber 10 is radially displaced outward and, in the assembled valve, is situated near an annular bulge 14 which is integral with the diaphragm disk 6.

Absent any lateral clamping or affixation, the diaphragm 6 is freely positioned in an annular space 16 provided between the two tube hookup housings 2 and 4, The dimensions of diameter and height of this annular space 16 are selected in a manner that, radially outside the annular bulge 14, said space 16 forms bypass channels 18. These bypass channels 18 connect the intake chamber 10 to an outlet chamber 22 communicating with the outlet channel 24 in the second tube hookup housing 4 when the diaphragm disk 6 is lifted from the valve seat 12 by a corresponding pressure in the intake chamber 10.

Supports 20 projecting in the direction of the diaphragm disk 6 are provided in a concentric, central zone of the outlet chamber 22, the ends of said supports resting against said disk and being dimensioned in a way to pretension, i.e. bias, the diaphragm disk 6 toward the valve seat 12.

A structure denoted generally by 28 is provided at least on the side of the diaphragm disk 6 facing the supports 20, which by engaging the ends 21 of the supports 20 precludes the diaphragm disk from displacing.

As shown, the supports 20 are positioned concentrically and circularly, the structure 28 in the preferred embodiment being a ring 30 integral with the diaphragm disk 6, as a result of which said disk is kept in place by the ends 21 of the supports 20 in nearly interlocking manner and concentrically in the annular space 16.

As shown by the appended Figures, the preferred embodiment comprises a structure 28 respectively 28a or ring 30 both on the top side 32 and the bottom side 34 of the diaphragm 6 to eliminate the requirement of any particular orientation of said disk 6 when assembling the check valve 1.

Because the bypass channels 18 function as the apertures used in the known art's diaphragm disk, the diaphragm disk 6 of the present invention is continuous and devoid of any apertures within the annular bulge 14.

The appended Figures moreover show that the diaphragm disk 6 is comparatively thick within the zone 36 of the annular bulge 14 to assure the desired bias toward the valve seat 12 without recourse to laterally clamping the said disk. The thickness of the diaphragm disk 6 within the zone of the annular bulge 14 is about 0.5 to 0.18 times the size of the cross-sectional diameter of the annular bulge 14.

As regards the preferred embodiment of the present invention of the check valve 1 shown in the Figures, generally stated, the ends 21 of the supports 20 engaging the diaphragm disk 6 and the structure 28, 28a respectively ring 30 are designed complementary to the cross-section of the structures 28, 28a. In the shown embodiment mode, the ring 30 is having a triangular cross-section, the supports 20 being fitted with matching inclined surfaces 38 resting against the outer legs of said triangular cross-section.

The above discussed check valve 1 of the shown embodiment mode operates at intake pressures of about 1.5 to 5.0 psi and allows pressure differentials up to 100 psi across the valve. At a given flow rate, the pressure loss across the valve is markedly low.

All features and advantages explicit in and implicit from the specification, claims and drawings of the present invention, including design details and spatial configurations, may be construed being inventive per se or in arbitrary combinations.

### LIST OF REFERENCES

1 check valve

2 first tube hookup housing

4 second hookup housing

6 diaphragm disk

8 intake passage

10 intake chamber

12 valve seat

14 annular bulge

16 annular space

18 bypass channel

20 support

21 end of 20

22 outlet chamber

24 outlet passage

28, 28a structures

30 ring

32 top side of 6

34 bottom side of 6

36 zone within 14

38 inclined surfaces

## Claims

1. A check valve (1) in particular for medical purposes, comprising a first tube hookup housing (2) and a second tube hookup housing (4) and a diaphragm disk (6) of a flexible plastic which is positioned between said two housings and which in the case of over-pressure in an intake passage (8) in the first tube hookup housing (2) is lifted off an annular valve seat (12) enclosing an intake chamber (10) connected to the intake passage (8) and which in the case of over-pressure in an outlet passage (24) can be pressed reliably and in minimal time onto the valve seat (12), wherein the intake chamber (10) is radially substantially enlarged with respect to the intake passage (8) and the valve seat (12) encloses the intake chamber (10) the diaphragm disk (6) is positioned freely in an annular space (16) between the tube hookup housings (2, 4), in that the annular space (16) forms bypass channels (18) radially outside the disk (6), said bypass channels connecting the intake chamber (10) to an outlet chamber (22) communicating with the outlet passage (24) when the diaphragm disk (12) has been lifted off the valve seat (12), supports (20) are positioned concentrically in the central zone of the outlet chamber (22) which and bias the membrane disk (6) toward the valve seat (12) and in that at least one or more structures (28, 28a) are provided at least on the side of the diaphragm disk (6) facing said supports (20), said structures, by engaging the ends (21) of the supports (20), precluding the diaphragm disk (6) from displacing, **characterized in that** said diaphragm disk (6) is provided at its outer periphery with an annular bulge (14) outside and near the valve seat.

2. Check valve as claimed in claim 1, **characterized in that** the supports (20) are positioned in a concentric, circular manner and **in that** the structures (28, 28a) are configured as an integral projecting ring (30) on the diaphragm disk (6).

3. Check valve as claimed in either of claims 1 and 2, **characterized in that** identical structures (28, 28a) are provided on the top side (32) and the bottom side (34) of the diaphragm disk (6).

4. Check valve as claimed in any one of the above claims, **characterized in that** the diaphragm disk (6) is continuous, devoid of apertures, within the annular bulge (14).

5. Check valve as claimed in any one of the above claims, **characterized in that** the diaphragm disk (6) is comparatively thick in the zone (36) within the annular bulge (14).

6. Check valve as claimed in claim 5, **characterized in that** the thickness of the diaphragm disk (6) in the zone (36) within the annular bulge (14) is approximately 0.5 to 0.8 times the size of the cross-sectional diameter of this annular bulge (14).

7. Check valve as claimed in one of the above claims, **characterized in that** the ends (21) of the supports (20) engaging the diaphragm disk (6) and the structure (28) respectively ring (30) are designed complementary to the cross-section of the structures (28, 28a).

8. Check valve as claimed in claim 7, **characterized in that** the ring (30) is having a triangular cross-section and that matching inclined surfaces (38) are provided on the supports (20).

## Patentansprüche

1. Rückschlagventil (1), insbesondere für medizinische Zwecke, mit einem ersten Schlauchanschlussgehäuse (2) und einem zweiten Schlauchanschlussgehäuse (4) und einer zwischen den beiden Gehäusen angeordneten Membranscheibe (6) aus flexiblem Kunststoff, welche bei Überdruck in einem Eingangsdurchlass (8) in dem ersten Schlauchanschlussgehäuse (2) von einem ringförmigen, einen mit dem Eingangsdurchlass (8) verbundenen Einlassraum (10) umgebenden Ventilsitz (12) abgehoben wird und die bei Überdruck in einem Ausgangsdurchlass (24) zuverlässig und unter minimaler Zeit auf dem Ventilsitz (12) andrückbar ist, wobei der Einlassraum (10) bezüglich des Eingangsdurchlasses (8) radial im Wesentlichen vergrößert ist und der Ventilsitz (12) den Einlassraum (10) umgibt, die Membranscheibe (6) in einem Ringraum (16) zwischen den Schlauchanschlussgehäusen (2, 4) frei positioniert ist, der Ringraum (16) radial außerhalb der Scheibe (6) Bypasskanäle (18) bildet, wobei die Bypasskanäle den Einlassraum (10) mit einem Auslassraum (22) verbinden, der mit einem Auslassdurchgang (24) in Verbindung steht, wenn die Membranscheibe (12) von dem Ventilsitz (12) abgehoben worden ist, Stützen (20) konzentrisch in dem mittleren Bereich des Auslassraums (22) positioniert sind und die Membranscheibe (6) zu dem Ventilsitz (12) vorspannen, und mindestens eine oder mehrere Strukturen (28, 28a) zumindest auf der Seite der Membranscheibe (6), die den Stützen (20) zugekehrt ist, vorgesehen sind, wobei die Strukturen durch Ineingriffnahme der Enden (21) der Stützen (20) eine Verschiebung der Membranscheibe (6) verhindern, **dadurch gekennzeichnet, dass** die Membranscheibe (6) an ihrem Außenumfang mit einer ringförmigen Ausbuchtung (13) außerhalb und in der Nähe des Ventilsitzes versehen ist.

2. Rückschlagventil nach Anspruch 1, **dadurch gekennzeichnet, dass** die Stützen (20) konzentrisch und kreisförmig positioniert sind und dass die Strukturen (28, 28a) als ein integraler vorragender Ring (30) an der Membranscheibe (6) konfiguriert sind.

3. Rückschlagventil nach einem der Ansprüche 1 und 2, **dadurch gekennzeichnet, dass** identische Strukturen (28, 28a) an der Oberseite (32) und der Unterseite (34) der Membranscheibe (6) vorgesehen sind.

4. Rückschlagventil nach einem der obigen Ansprüche, **dadurch gekennzeichnet, dass** die Membranscheibe (6) in der ringförmigen Ausbuchtung (14) durchgehend ohne Öffnungen ausgebildet ist.

5. Rückschlagventil nach einem der obigen Ansprüche, **dadurch gekennzeichnet, dass** die Membranscheibe (6) in dem Bereich (36) in der ringförmigen Ausbuchtung (14) vergleichsweise dick ist.

6. Rückschlagventil nach Anspruch 5, **dadurch gekennzeichnet, dass** die Dicke der Membranscheibe (6) in dem Bereich (36) in der ringförmigen Ausbuchtung (14) ca. das 0,5-Fache bis 0,8-Fache der Größe des Querschnittsdurchmessers dieser ringförmigen Ausbuchtung (14) beträgt.

7. Rückschlagventil nach einem der obigen Ansprüche, **dadurch gekennzeichnet, dass** die Enden (21) der Stützen (20), die mit der Membranscheibe (6) und der Struktur (28) bzw. dem Ring (30) in Eingriff stehen, komplementär zu dem Querschnitt der Strukturen (28, 28a) ausgeführt sind.

8. Rückschlagventil nach Anspruch 7, **dadurch gekennzeichnet, dass** der Ring (30) einen dreieckigen Querschnitt aufweist und dass entsprechend geneigte Flächen (38) an den Stützen (20) vorgesehen sind.

## Revendications

1. Clapet anti-retour (1), en particulier à usage médical, comprenant un premier boîtier d'accrochage tubulaire (2) et un deuxième boîtier d'accrochage tubulaire (4) et un disque à diaphragme (6) en plastique flexible, qui est positionné entre lesdits deux boîtiers et qui, dans le cas d'une surpression dans un passage d'admission (8) dans le premier boîtier d'accrochage tubulaire (2), est soulevé d'un siège de clapet annulaire (12) entourant une chambre d'admission (10) connectée au passage d'admission (8) et qui, dans le cas d'une surpression dans le passage de sortie (24), peut être pressé de manière fiable et dans un temps minimal sur le siège de clapet (12), la chambre d'admission (10) étant radialement substantiellement élargie par rapport au passage d'admission (8) et le siège de clapet (12) renfermant la chambre d'admission (10), le disque à diaphragme (6) étant positionné librement dans un espace annulaire (16) entre les boîtiers d'accrochage tubulaires (2, 4), l'espace annulaire (16) formant des canaux de dérivation (18) radialement à l'extérieur du disque (6), lesdits canaux de dérivation connectant la chambre d'admission (10) à une chambre de sortie (22) communiquant avec le passage de sortie (24) lorsque le disque à diaphragme (6) a été soulevé du siège de clapet (12), des supports (20) étant positionnés concentriquement dans la zone centrale de la chambre de sortie (22) et sollicitant le disque à diaphragme (6) vers le siège de clapet (12), et au moins une ou plusieurs structures (28, 28a) étant prévues au moins sur le côté du disque à diaphragme (6) faisant face auxdits supports (20), lesdites structures, en engageant les extrémités (21) des supports (20), empêchant le disque à diaphragme (6) de se déplacer, **caractérisé en ce que** ledit disque à diaphragme (6) est pourvu, sur sa périphérie extérieure, d'un renflement annulaire (14) à l'extérieur et à proximité du siège de clapet.

2. Clapet anti-retour selon la revendication 1, **caractérisé en ce que** les supports (20) sont positionnés de manière concentrique circulaire et **en ce que** les structures (28, 28a) sont configurées sous forme de bague saillante intégrale (30) sur le disque à diaphragme (6).

3. Clapet anti-retour selon la revendication 1 ou 2, **caractérisé en ce que** des structures identiques (28, 28a) sont prévues sur le côté supérieur (32) et le côté inférieur (34) du disque à diaphragme (6).

4. Clapet anti-retour selon l'une quelconque des revendications précédentes, **caractérisé en ce que** le disque à diaphragme (6) est continu, sans ouvertures, dans le renflement annulaire (14).

5. Clapet anti-retour selon l'une quelconque des revendications précédentes, **caractérisé en ce que** le disque à diaphragme (6) est relativement épais dans la zone (36) dans le renflement annulaire (14).

6. Clapet anti-retour selon la revendication 5, **caractérisé en ce que** l'épaisseur du disque à diaphragme (6) dans la zone (36) dans le renflement annulaire (14) est d'environ 0,5 à 0,8 fois la taille du diamètre en section transversale de ce renflement annulaire (14).

7. Clapet anti-retour selon l'une quelconque des revendications précédentes, **caractérisé en ce que** les extrémités (21) des supports (20) engageant le disque à diaphragme (6) et la structure (28), respectivement la bague (30), sont conçues de manière complémentaire à la section transversale des structures (28, 28a).

8. Clapet anti-retour selon la revendication 7, **caractérisé en ce que** la bague (30) a une section transversale triangulaire et **en ce que** des surfaces inclinées correspondantes (38) sont prévues sur les supports (20).
